# EUROPÄISCHE PATENTANMELDUNG

(11) **EP 3 189 769 A1**
(43) Veröffentlichungstag der Anmeldung: **12.07.2017**
(21) Anmeldenummer: 16206701.1
(22) Anmeldetag: 23.12.2016
(51) Int. Cl.: A61B 1/00, A61B 1/12

(54) **VERFAHREN, VORRICHTUNG UND SYSTEM ZUM AUFBEREITEN VON ENDOSKOPEN**

(30) Priorität: 05.01.2016 DE 102016200037
(71) Anmelder: OLYMPUS Winter & Ibe GmbH, 22045 Hamburg (DE)
(72) Erfinder: LENGSFELD, Michael, 22179 Hamburg (DE); THATE, Henning, 22417 Hamburg (DE)
(74) Vertreter: Seemann & Partner Patentanwälte mbB

(57) **Zusammenfassung**

Die Erfindung betrifft ein Verfahren zum Aufbereiten eines, insbesondere flexiblen, Endoskops (2), eine Vorrichtung (20) zum Beaufschlagen wenigstens eines aufzubereitenden, insbesondere flexiblen, Endoskops (2) mit Druckluft, die Teil eines Systems zum Aufbereiten wenigstens eines, insbesondere flexiblen, Endoskops ist, sowie ein System (10) zur Aufbereitung wenigstens eines, insbesondere flexiblen, Endoskops (2), umfassend eine Aufbereitungseinheit (30) und eine Vorrichtung (20) zum Beaufschlagen wenigstens eines aufzubereitenden, insbesondere flexiblen, Endoskops (2) mit Druckluft.

Erfindungsgemäß wird während einer Aufbereitung (70) eine Differenz (84, ΔV) zwischen hinzugegebener und abgelassener Druckluft erfasst und bei einem Überwiegen des Ablassens von Druckluft gegenüber dem Hinzugeben von Druckluft über einen längeren Zeitraum während der Aufbereitung (70) und/oder nach einer Elimination eines Einflusses von Temperaturschwankungen eine Undichtigkeit des Endoskops (2) aufgrund eines Eindringens von Spülflüssigkeit aus einem zu spülenden Kanal in das Endoskop (2) festgestellt.

## Beschreibung

Die Erfindung betrifft ein Verfahren zum Aufbereiten eines, insbesondere flexiblen, Endoskops, eine Vorrichtung zum Beaufschlagen wenigstens eines aufzubereitenden, insbesondere flexiblen, Endoskops mit Druckluft sowie ein System zur Aufbereitung wenigstens eines, insbesondere flexiblen, Endoskops, umfassend eine Aufbereitungseinheit und eine Vorrichtung zum Beaufschlagen wenigstens eines aufzubereitenden, insbesondere flexiblen, Endoskops mit einem Luftdruck.

Zur Aufbereitung von, insbesondere flexiblen, Endoskopen werden üblicherweise Reinigungs- und Desinfektionsgeräte (RDG-E) eingesetzt, die auf einem chemisch-thermischen Aufbereitungsprozess basieren. Um die Endoskope durch die Reinigungsflüssigkeit nicht zu beschädigen, ist es wichtig, dass die Hülle des Endoskops vollständig intakt ist und somit Reinigungsflüssigkeit nicht in das Innere des Endoskops eindringen kann. Dies wird im RDG-E vor dem ersten Wassereinlauf gemäß DIN EN 15883 automatisch überprüft, indem das Endoskop über eine entsprechende Schnittstelle auf einen Überdruck aufgepumpt wird. Anschließend wird der Druckabfall überwacht, wobei aus einem Druckabfall auf eine Leckage in dem Endoskop geschlossen werden kann. Bei fehlgeschlagener Prüfung bzw. zu Prozessende, also zum Ende des Reinigungs- und Desinfektionsprozesses, wird das Endoskop wieder teilweise entlüftet. Die Aufbereitung selbst erfolgt bei geringerem Überdruck im Endoskop.

Entsprechende Systeme sind aus dem RDG-E-System der Anmelderin bekannt, die als ETD-Serie verkauft wird, derzeit in der vierten Generation als ETD4. Diese Systeme umfassen einen sogenannten "Leak Tester", der den Dichtigkeitstest durchführt. Dazu wird im ETD4-System der Anmelderin ein Endoskop initial nach Programmstart zur Dichtigkeitsprüfung mit einem Überdruck von 285 mbar gegenüber dem Umgebungsdruck ("ambient pressure", AP) aufgepumpt und der Überdruck nach dem Dichtigkeitstest auf ca. 150 mbar über Umgebungsdruck reduziert. Dieser Druck wird während der Aufbereitung gehalten und kontinuierlich überwacht, auch um das Eindringen von Wasser von außen zu verhindern. Die Spülzyklen mit kaltem und warmen Wasser führen zu teils starken Temperaturänderungen, die dazu führen, dass sich der Druck im Endoskop ändert. Dies wird durch das Zugeben oder Ablassen von Druckluft in das Endoskop bzw. aus dem Endoskop heraus kompensiert.

Bei der Verwendung der Endoskope in der Endoskopie treten vereinzelt Schäden auf, bei denen Endoskope von Biopsiekanälen her punktiert werden. Es entsteht eine Mikroperforation, die bei einem gemäß DIN EN 15883 durchgeführten Test unter Umständen nicht erkannt wird, weil die Undichtigkeit eine Richtungsabhängigkeit aufweisen kann, entsprechend einem Rückschlagventil. Bei der nachfolgenden Aufbereitung wird Spül- und Klarwasser mit einem deutlich größeren Druck als 150 mbar durch die zu spülenden Kanäle geleitet. Dadurch kann dann ein Übertrag von Wasser aus der Kanalspülung in den zu schützenden Bereich des Endoskops erfolgen, was zu einer Druckerhöhung im Endoskop über 150 mbar hinaus führt. Der Leak Tester reagiert darauf mit einem Ablassen von Druckluft aus dem Endoskop, um den Überdruck von 150 mbar zu halten. Dadurch kann Wasser über den Weg der Entlüftung in die Verschlauchung eindringen und schlussendlich in den Leak Tester eingetragen werden und diesen schlimmstenfalls zerstören.

Der vorliegenden Erfindung liegt demgegenüber die Aufgabe zugrunde, eine Aufbereitung von Endoskopen unter Vermeidung der bislang anwesenden Probleme zu verbessern.

Diese Aufgabe wird durch ein Verfahren zum Aufbereiten eines, insbesondere flexiblen, Endoskops gelöst, wobei ein aufzubereitendes Endoskop zur Spülung von wenigstens einem zu spülenden Kanal mit einem Spülschlauch verbunden wird und zur Dichtigkeitsprüfung des Endoskops mit einem Druckluftschlauch druckluftdicht verbunden wird, der mit einer Druckluftquelle verbindbar oder verbunden ist, wobei zunächst ein direkter pneumatischer Dichtigkeitstest durchgeführt wird, bei dem das Endoskop durch den Druckluftschlauch bis zu einem vorgegebenen oder vorgebbaren ersten Überdruckniveau mit Druckluft beaufschlagt wird, während einer direkten pneumatischen Dichtigkeitsmessung ohne weitere Druckluftzufuhr ein Verlauf eines Luftdrucks im Endoskop gemessen wird und aus einem Verlauf des Luftdrucks während der direkten pneumatischen Dichtigkeitsmessung bestimmt wird, ob das Endoskop den pneumatischen Dichtigkeitstest bestanden hat, wobei nach Bestehen des pneumatischen Dichtigkeitstests ein Teil der Druckluft bis zum Erreichen eines zweiten Überdruckniveaus aus dem Endoskop abgelassen wird, das unterhalb des ersten Überdruckniveaus liegt, und anschließend eine Aufbereitung des Endoskops durchgeführt wird, bei der Spülflüssigkeit unter Druck durch den wenigstens einen zu spülenden Kanal geleitet wird, wobei der pneumatische Überdruck im Endoskop während der Aufbereitung innerhalb eines vorgebbaren oder vorgegebenen Luftdruckbereichs um das zweite Überdruckniveau herum durch Hinzugeben oder Ablassen von Druckluft nachgeführt wird, das dadurch weitergebildet ist, dass während der Aufbereitung eine Differenz zwischen hinzugegebener und abgelassener Druckluft erfasst wird und bei einem Überwiegen des Ablassens von Druckluft gegenüber dem Hinzugeben von Druckluft über einen längeren Zeitraum während der Aufbereitung und/oder nach einer Elimination eines Einflusses von Temperaturschwankungen eine Undichtigkeit des Endoskops aufgrund eines Eindringens von Spülflüssigkeit aus einem zu spülenden Kanal in das Endoskop festgestellt wird.

Unter einer Spülflüssigkeit wird im Rahmen der vorliegenden Erfindung jede Flüssigkeit verstanden, die während der Aufbereitung durch zu spülende Kanäle des Endoskops geleitet werden, mit oder ohne Beaufschlagung von Reinigungschemikalien und unabhängig von der Temperatur der Flüssigkeit.

Das bekannte Dichtigkeitstestverfahren nach ISO EN 15883 wird somit erfindungsgemäß ergänzt durch eine indirekte Überwachung des trocken zu haltenden Lumens des Endoskops während der Aufbereitung. Grundsätzlich ist ein Netto-Verlust an Druckluft aus dem Endoskop während der Aufbereitung ein Indikator für ein schrumpfendes Volumen aufgrund eines Eindringens von Flüssigkeit durch eine Punktion in der Scheidewand zwischen beispielsweise einem gerade gespülten Biopsiekanal und einem eigentlich trocken zu haltenden optischen Kanal. Dieser Netto-Verlust ist allerdings überlagert durch die Regelfunktion des Leak Testers, der während der Aufbereitung ständig Druckluft hinzugibt oder ablässt, um in Reaktion auf teils starke Temperaturschwankungen bei der Aufbereitung den Überdruck im Wesentlichen konstant bei dem zweiten Überdruckniveau zu halten. Eine direkte Messung ist daher nicht möglich.

Um diese Überlagerung auszuschalten, sind erfindungsgemäß zwei Lösungen vorgesehen, die getrennt verwendet werden können, aber auch gegebenenfalls gemeinsam verwirklicht sein können. Eine erste Lösung besteht darin, den Netto-Verlust oder Netto-Gewinn an Druckluft nur zwischen Zeitpunkten der Aufbereitung zu berechnen, an denen die gemessene Temperatur die gleiche oder annähernd die gleiche ist. Vorzugsweise ist in diesem Fall außerdem der Überdruck der gleiche oder zumindest annähernd der gleiche, beispielsweise innerhalb von wenigen mbar, beispielsweise bis zu 5 mbar. Gerade in Phasen starker Temperaturänderungen wird es der Steuervorrichtung in vielen Fällen nicht gelingen, den Überdruck exakt konstant beim zweiten Überdruckniveau zu halten. Die Vergleichbarkeit der Zustände in Bezug auf Temperatur und gegebenenfalls auch Druck sorgt in diesem Fall dafür, dass Korrekturen an der Messung der Druckluftzu- und -abfuhr unnötig sind.

In diesem Fall wird vorteilhafterweise zur Eliminierung des Einflusses von Temperaturschwankungen die Menge an Druckluft berechnet, die zwischen zwei unterschiedlichen Zeitpunkten hinzugekommen oder abgelassen worden ist, bei denen Temperaturen gemessen wurde, deren Unterschied weniger als 15 °C beträgt, insbesondere weniger als 3 °C.

Alternativ oder zusätzlich hierzu kann erfindungsgemäß der Einfluss von Temperaturschwankungen, gegebenenfalls vorzugsweise außerdem auch von Schwankungen im gemessenen Überdruck, eliminiert bzw. herausgerechnet werden. So kann in einer vorteilhaften Ausführung des Verfahrens ein temperatur- und/oder druckabhängiger Erwartungswert für die Luftmenge bzw. die Druckluftzu- und - abfuhr gebildet werden, der mit der gemessenen Druckluftzu- und - abfuhr verglichen wird. Eine vorteilhafte Ausbildung des Verfahrens sieht in diesem Fall vor, dass zur Eliminierung des Einflusses von Temperaturschwankungen die Menge an hinzugekommener oder abgelassener Druckluft um den Effekt von Temperaturschwankungen korrigiert wird, wobei zur Korrektur insbesondere eine, insbesondere lineare oder quadratische, Musterfunktion verwendet wird, die anhand von in der vorausgegangenen Dichtigkeitsprüfung ermittelten Faktoren an das Endoskop angepasst wird.

So ist es vorzugsweise vorgesehen, beim Aufpumpen des Endoskops vom Umgebungsdruck auf das erste Überdruckniveau die funktionelle Abhängigkeit des Überdrucks vom zugeführten Druckluftvolumen zu bestimmen. Da das Endoskop ein näherungsweise konstantes Volumen aufweist und die verwendete Druckluft wenigstens näherungsweise die thermodynamischen Eigenschaften eines idealen Gases aufweist, verändert sich bei konstantem Volumen der Luftdruck im Wesentlichen proportional zur Temperatur, bezogen auf den absoluten Nullpunkt bei 0 K. Die zunächst gemessene Abhängigkeit des Überdrucks vom zugeführten Luftvolumen kann auch nichtlineare Terme, beispielsweise quadratische oder höhere Terme, beinhalten, die beispielsweise von einer leichten Ausdehnung des Endoskops bei steigendem Innendruck herrühren können.

Eine Kombination beider vorgenannter Lösungen ist besonders vorteilhaft, weil gerade bei kleinen Unterschieden in den Temperaturen und Drücken zu unterschiedlichen Messzeitpunkten eine Korrektur anhand der gemessenen Temperaturen bzw. Drücken klein ist und zu einer sehr sicheren Messung führt. Solche kleinen Korrekturen verbessern die Messung, ohne ihrerseits größere Unsicherheiten einzuführen.

Vorteilhafterweise wird die festgestellte Undichtigkeit des Endoskops mit einem Grenzwert oder mehreren vorbestimmten oder vorbestimmbaren Grenzwerten verglichen. Dies ermöglicht die Einleitung geeigneter Maßnahmen bei Überschreitung des jeweiligen Grenzwerts. So wird vorzugsweise bei einem Überschreiten eines ersten Grenzwerts der Überdruck im Endoskop auf oberhalb des zweiten Überdruckniveaus erhöht, um zu erkennen, ob wirklich ein Eindringen von Wasser vorliegt. Dies ist ziemlich wahrscheinlich der Fall, falls die Tendenz des Druckluftverlusts durch die Überdruckerhöhung nicht gestoppt wird. Alternativ oder zusätzlich wird bei einem Überschreiten eines zweiten Grenzwerts die Aufbereitung abgebrochen und insbesondere ein Defekt des Endoskops festgestellt, wobei insbesondere der zweite Grenzwert höher als der erste Grenzwert ist, falls ein zuvor genannter erster Grenzwert überprüft wird.

In zum erfindungsgemäßen Verfahren paralleler Weise wird die der Erfindung zugrunde liegende Aufgabe auch durch eine Vorrichtung zum Beaufschlagen wenigstens eines aufzubereitenden, insbesondere flexiblen, Endoskops mit Druckluft, die Teil eines Systems zum Aufbereiten wenigstens eines, insbesondere flexiblen, Endoskops ist, gelöst, umfassend eine Druckluftquelle, einen an ein Endoskop druckluftdicht anschließbaren Druckluftschlauch, eine mit dem Druckluftschlauch direkt oder indirekt pneumatisch verbindbare Luftdruckmesseinheit und eine mit der Druckluftquelle und der Luftdruckmesseinheit verbundene Steuereinheit, die ausgebildet und eingerichtet ist, in einem pneumatischen Dichtigkeitstest das Endoskop durch den Druckluftschlauch bis zu einem vorgegebenen oder vorgebbaren ersten Überdruckniveau mit Druckluft zu beaufschlagen, während einer pneumatischen Dichtigkeitsmessung ohne weitere Druckluftzufuhr einen Verlauf des Luftdrucks im Endoskop zu erfassen und aus einem Verlauf des Luftdrucks während der pneumatischen Dichtigkeitsmessung zu bestimmen, ob das Endoskop den pneumatischen Dichtigkeitstest bestanden hat, nach Bestehen des pneumatischen Dichtigkeitstests einen Teil der Druckluft bis zum Erreichen eines zweiten Überdruckniveaus aus dem Endoskop abzulassen, das unterhalb des ersten Überdruckniveaus liegt, und während einer Aufbereitung des Endoskops, bei der Spülflüssigkeit unter Druck durch den wenigstens einen zu spülenden Kanal geleitet wird, den pneumatischen Überdruck im Endoskop innerhalb eines vorgebbaren oder vorgegebenen Luftdruckbereichs um das zweite Überdruckniveau herum durch Hinzugeben oder Ablassen von Druckluft nachzuführen, wobei die Vorrichtung dadurch weitergebildet ist, dass die Steuereinrichtung zusätzlich ausgebildet und eingerichtet ist, während der Aufbereitung eine Differenz zwischen hinzugegebener und abgelassener Druckluft zu erfassen und bei einem Überwiegen des Ablassens von Druckluft gegenüber dem Hinzugeben von Druckluft über einen längeren Zeitraum während der Aufbereitung und/oder nach einer Elimination des Einflusses von Temperaturschwankungen eine Undichtigkeit des Endoskops aufgrund Eindringens von Spülflüssigkeit aus einem zu spülenden Kanal in das Endoskop festzustellen.

Damit teilt die erfindungsgemäße Vorrichtung alle Vorteile, Eigenschaften und Merkmale des zuvor beschriebenen erfindungsgemäßen Verfahrens. Die Steuervorrichtung ist vorteilhafterweise auch ausgebildet und eingerichtet, ein zuvor beschriebenes erfindungsgemäßes Verfahren durchzuführen. Sie ist weiter vorzugsweise auch eine Steuervorrichtung einer Aufbereitungseinheit des Systems zum Aufbereiten wenigstens eines, insbesondere flexiblen, Endoskops.

Die der Erfindung zugrunde liegende Aufgabe wird in gleicher Weise auch durch ein System zur Aufbereitung wenigstens eines, insbesondere flexiblen, Endoskops, umfassend eine Aufbereitungseinheit und eine zuvor beschriebene erfindungsgemäße Vorrichtung zum Beaufschlagen wenigstens eines aufzubereitenden, insbesondere flexiblen, Endoskops mit Druckluft gelöst. Damit teilt auch das System alle Vorteile, Eigenschaften und Merkmale des zuvor beschriebenen erfindungsgemäßen Verfahrens und der zuvor beschriebenen erfindungsgemäßen Vorrichtung.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines erfindungsgemäßen Reinigungs- und Desinfektionsgerät und
- Fig. 2: eine schematische Darstellung eines erfindungsgemäßen Verfahrens.
- Fig. 3a) bis 3c): beispielhafte Verläufe von Überdruck, Temperatur und Druckluftzu- und -abfuhren bei einer Aufbereitung.

In den Zeichnungen sind jeweils gleiche oder gleichartige Elemente und/oder Teile mit denselben Bezugsziffern versehen, so dass von einer erneuten Vorstellung jeweils abgesehen wird.

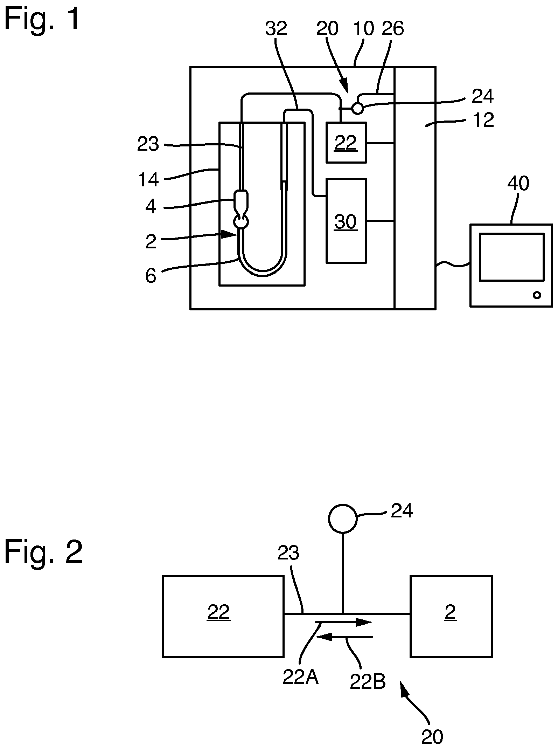

Fig. 1 zeigt eine schematische Darstellung eines erfindungsgemäßen Aufbereitungssystems 10, in dessen Reinigungskammer 14 ein flexibles Endoskop 2 angeordnet ist, das bereit ist, gereinigt und desinfiziert zu werden. Das Endoskop 2 umfasst einen Griff 4 sowie einen flexiblen Schaft 6, die beide an Anschlüsse des Aufbereitungssystems 10 angeschlossen sind.

Der Schaft 6 des Endoskops 2 ist über einen Anschluss mit einer Aufbereitungsvorrichtung 30 des Aufbereitungssystems 10 verbunden, während der Handgriff 4 über einen Druckluftschlauch 23 mit einer Druckluftquelle 22, beispielsweise einem Kompressor, mit der Vorrichtung 20 zur Druckluftbeaufschlagung, kurz "Leakage Tester" genannt, verbunden ist. Diese ist, ebenso wie die Aufbereitungsvorrichtung 30, mit einer Steuervorrichtung 12 des Aufbereitungssystems 10 verbunden, die somit auch eine Steuervorrichtung für die Vorrichtung 20 zur Druckluftbeaufschlagung darstellt.

Ausgangs der Druckluftquelle 22 ist ein Luftdrucksensor 24 an den Druckluftschlauch 23 angeschlossen, der den Überdruck der Luft in dem Druckluftschlauch 23 über dem Umgebungsdruck ("ambient pressure", "AP") misst und entsprechende Signale über eine Signalleitung 26 ebenfalls an die Steuervorrichtung 12 des Aufbereitungssystems 10 übermittelt. Die Steuervorrichtung 12 ist außerdem mit einer Anzeigevorrichtung 40 verbunden, mittels der die Steuervorrichtung 12 von außen beeinflusst werden kann und umgekehrt Daten aus der Steuervorrichtung 12, beispielsweise Überdruckmessdaten von dem Luftdrucksensor 24 und/oder Prozessdaten, angezeigt werden können.

Fig. 2 zeigt schematisch einen Teil der Vorrichtung 20 zur Druckluftbeaufschlagung mit einer Druckluftquelle 22, die über einen Druckluftschlauch 23 mit einem nur schematisch angedeuteten Endoskop 2 verbunden ist und Druckluft in das Endoskop 2 pumpen kann und es auch wieder auslassen kann. Entsprechend ist mit jeweils einem Pfeil ein Volumenstrom 22A von Druckluft beim Aufpumpen und ein Volumenstrom 22B beim Ablassen von Druckluft gezeigt, wobei der Volumenstrom 22A von der Druckluftquelle 22 zum Endoskop 2 hin verläuft, während der Volumenstrom 22B beim Ablassen von Druckluft vom Endoskop 2 in Richtung auf die Druckluftquelle 22 verläuft, die einen nicht dargestellten Auslass zum Auslassen der Druckluft aufweist. Im Falle einer Punktion kann es zum Eindringen von Spülflüssigkeit in das Endoskop 2 kommen, die beim Ablassen von Druckluft mit dem Volumenstrom 22B in die Druckluftquelle 22 geraten und diese beschädigen kann.

Mit dem Druckluftschlauch 23 ist ein Luftdrucksensor 24 verbunden, der den Druck der Druckluft im Druckluftschlauch 23 und im Endoskop 2 misst. Der Luftdrucksensor 24 kann alternativ auch am Endoskop 2 direkt messen oder am Ausgang der Druckluftquelle 22.

Nach Abschluss des initialen Dichtigkeitstests wird der Überdruck auf einem konstanten Niveau gehalten, beispielsweise 150 mbar über dem Umgebungsdruck ("AP + 150 mbar"). Wenn beispielsweise aufgrund einer Temperaturerhöhung der Druck am Luftdrucksensor 24 steigt, wird mit einem Volumenstrom 22B Druckluft abgelassen, bis sich der gewünschte Druck wieder einstellt. Sinkt aufgrund einer Abkühlung der Druck, so wird von der Druckluftquelle 22 mit einem Volumenstrom 22A wieder Druckluft in das Endoskop 2 gepumpt, bis der gewünschte Druck wieder erreicht wird. Diese Regelung erfolgt kontinuierlich.

Bei dem erfindungsgemäßen Verfahren wird die Menge des Volumenstroms 22A, 22B kontinuerlich ermittelt, so dass zu jedem Zeitpunkt bekannt ist, wieviel Luft sich nach Abzug des bekannten Luftvolumens im Druckluftschlauch 23 im Endoskop 2 befindet. Im Falle des Eindringens von Spülflüssigkeit in das Endoskop 2 nimmt die Menge an Luft tendenziell ab. Diese Abnahme wird erfindungsgemäß als Indikator für eine Undichtigkeit verwendet. Aufgrund der während der Aufbereitung immer wieder auftretenden Temperaturschwankungen sind hierzu allerdings Maßnahmen erforderlich, die den überlagernden Effekt der Temperaturschwankungen ausgleichen.

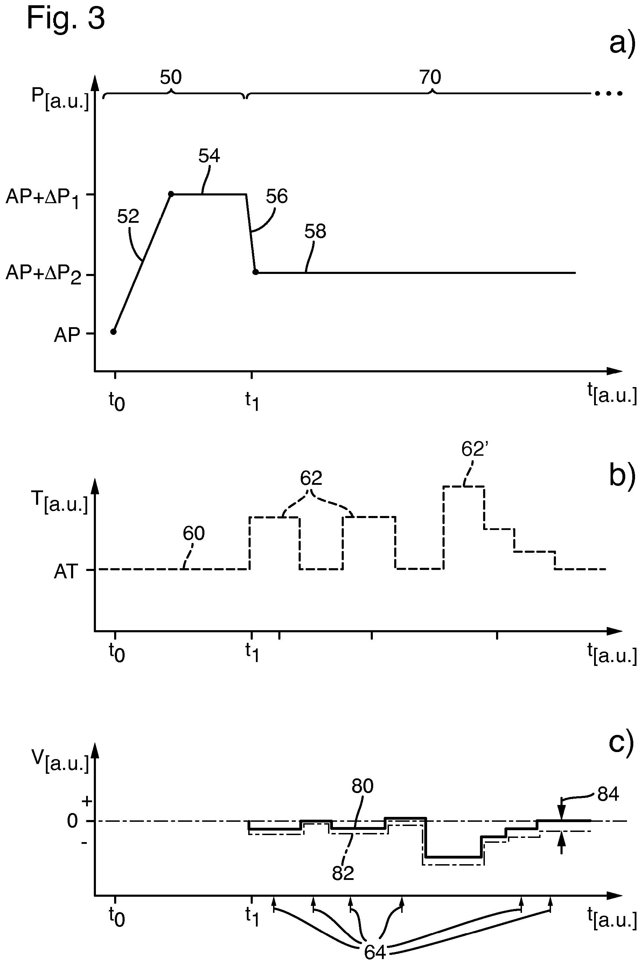

Fig. 3 zeigt beispielhafte Verläufe von Überdruck, Temperatur und Druckluftzu- und -abfuhren bei einer typischen Aufbereitung eines Endoskops.

Fig. 3a) zeigt den Verlauf des Überdrucks P in willkürlichen Einheiten ("arbitrary units", "a.u."). In der Phase der direkten pneumatischen Dichtigkeitsmessung 50 zwischen den Zeitpunkten t₀ und t₁ wird das Endoskop 2 mit Druckluft beaufschlagt (Schritt 52), so dass der Druck von Umgebungsdruck ("ambient pressure", "AP") auf ein erstes, hohes, Überdruckniveau AP+ΔP₁ steigt, wobei ΔP₁ beispielsweise, wie im ETD4-System der Anmelderin, 285 mbar betragen kann. Andere Werte sind ebenso möglich. In einer Messphase 54 wird keine weitere Druckluft zugeführt und der Verlauf des Drucks gemessen. Ist dieser konstant oder ist nur ein sehr geringer Verlust zu verzeichnen, so hat das Endoskop den direkten pneumatischen Dichtigkeitstest 50 bestanden und kann nachfolgend aufbereitet, also gereinigt und desinifiziert werden.

Die Abhängigkeit der aufsteigenden Kurve während der Beaufschlagung 52 des Endoskops 2 mit Druckluft vom Volumen der zugeführten Druckluft kann auch zur Kalibrierung einer Musterfunktion oder Korrekturfunktion für eine spätere erfindungsgemäße Messung gemessen und verwendet werden.

Vor oder zu Beginn der Aufbereitungsphase 70 wird eine Teilentleerung 56 des Endoskops 2 durchgeführt, bis der Druck im Endoskop 2 auf einem niedrigeren zweiten Überdruckniveau AP+ΔP₂ liegt, der im ETD4-System der Anmelderin beispielsweise 150 mbar über dem Umgebungsdruck beträgt. Auf bzw. bei diesem Niveau wird der Überdruck während der Aufbereitung 70 im Rahmen einer Druckluftnachführung 58 durch Regelung des Zugebens und Ablassens von Druckluft in das Endoskop 2 gehalten.

Die rein beispielhafte Temperaturkurve, ebenfalls in willkürlichen Einheiten, in Fig. 3b) setzt im Wesentlichen zu Beginn der Aufbereitung 70 an, zuvor verläuft sie flach (Bereich 60), beispielsweise auf dem Niveau der Umgebungstemperatur. In der Aufbereitung 70 weist sie mehrere Phasen erhöhter Temperatur auf, die sich mit Phasen niedriger Temperatur abwechseln. Hierbei handelt es sich um Spülgänge 62 mit Reinigungschemikalien und die anschließenden Spülgänge mit kaltem, klarem Wasser.

Fig. 3c) zeigt das Volumen im Endoskop 2 in willkürlichen Einheiten, wobei ein Netto-Zustrom zu positiven Werten ("+") und ein Netto-Abfluss zu negativen Werten ("-") führt, gemessen relativ zum Zeitpunkt t₁ zu Beginn der Aufbereitung 70. Es ist deutlich erkennbar, dass das Volumen V bzw. die Menge der Druckluft im Endoskop 2 sich umgekehrt proportional zur Temperatur verhält. Es sind zwei Kurven eingezeichnet, von denen die Kurve 80 den Verlauf ohne ein Leck und die Kurve 82 den Verlauf mit einem Leck darstellt, durch das Flüssigkeit in das Endoskop 2 eindringt. Dieses verdrängt Druckluft, was sich in einer Netto-Abnahme bzw. einer Differenz 84 niederschlägt, die im Verlaufe der Aufbereitung 70 immer weiter anwächst. Diese Differenz 84 kann mit Grenzwerten verglichen werden, um geeignete Maßnahmen zu ergreifen.

Die Kurve 80 in Fig. 3c) kann im Verlauf der Aufbereitung 70 als Musterantwort oder Korrekturantwort in Abhängigkeit der Temperaturkurve aus Fig. 3b) berechnet werden und mit der tatsächlich gemessenen Kurve 82 verglichen werden, um die Differenz 84 zu bilden und nachzuverfolgen. Alternativ oder zusätzlich können Messungen der Druckluftmenge V auch zu verschiedenen Zeitpunkten 64 geschehen, an denen die Bedingungen von Druck und/oder Temperatur vergleichbar sind. Dies betrifft zum einen die kalten Klarspülgänge und zum anderen Warmspülgänge mit gleichen Temperaturen. Hierdurch werden Modellunsicherheiten im Wesentlichen ausgeschlossen. Bei leichten Schwankungen, wie sie in der Nachführung des Drucks auftreten können, ist eine Korrektur der Messungen allerdings nach wie vor vorteilhaft, da sie die Vergleichbarkeit erhöhen, aufgrund der Geringfügigkeit der Korrekturen aber keine oder nur äußerst geringfügige eigene Unsicherheiten in die Messung einbringen.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Endoskop
- 4: Griff
- 6: flexibler Schaft
- 10: Aufbereitungssystem
- 12: Steuereinheit
- 14: Reinigungskammer
- 20: Vorrichtung zur Druckluftbeaufschlagung
- 22: Druckluftquelle
- 22A: Volumenstrom beim Hinzufügen von Druckluft
- 22B: Volumenstrom beim Ablassen von Druckluft
- 23: Druckluftschlauch
- 24: Luftdrucksensor
- 26: Signalleitung
- 30: Aufbereitungsvorrichtung
- 32: Spülschlauch
- 40: Anzeigeeinheit
- 50: direkte pneumatische Dichtigkeitsmessung
- 52: Beaufschlagen des Endoskops mit Druckluft
- 54: Messphase
- 56: Teilentleeren
- 58: Druckluftnachführung
- 60: Bereich ohne Temperaturschwankung
- 62: Spülgänge mit erhöhter Temperatur
- 64: mögliche Messzeitpunkte
- 70: Aufbereitung
- 80: Verlauf ohne Leck
- 82: Verlauf mit Leck
- 84: Differenz aufgrund eines Lecks
- AP: Umgebungsdruck
- ΔP+ΔP₁: erstes Überdruckniveau
- AP+ΔP₂: zweites Überdruckniveau
- AT: Umgebungstemperatur
- P: Druck
- t: Zeit
- ΔV: Volumen zu- und abgeführter Druckluft

## Patentansprüche

1. Verfahren zum Aufbereiten eines, insbesondere flexiblen, Endoskops (2), wobei ein aufzubereitendes Endoskop (2) zur Spülung von wenigstens einem zu spülenden Kanal mit einem Spülschlauch (32) verbunden wird und zur Dichtigkeitsprüfung des Endoskops (2) mit einem Druckluftschlauch (23) druckluftdicht verbunden wird, der mit einer Druckluftquelle (22) verbindbar oder verbunden ist, wobei zunächst ein direkter pneumatischer Dichtigkeitstest durchgeführt wird, bei dem das Endoskop (2) durch den Druckluftschlauch (23) bis zu einem vorgegebenen oder vorgebbaren ersten Überdruckniveau (ΔP+ΔP₁) mit Druckluft beaufschlagt wird, während einer direkten pneumatischen Dichtigkeitsmessung (50) ohne weitere Druckluftzufuhr ein Verlauf eines Luftdrucks (P) im Endoskop (2) gemessen wird und aus einem Verlauf des Luftdrucks (P) während der direkten pneumatischen Dichtigkeitsmessung (50) bestimmt wird, ob das Endoskop (2) den pneumatischen Dichtigkeitstest bestanden hat, wobei nach Bestehen des pneumatischen Dichtigkeitstests ein Teil der Druckluft bis zum Erreichen eines zweiten Überdruckniveaus (AP+ΔP₂) aus dem Endoskop (2) abgelassen wird, das unterhalb des ersten Überdruckniveaus (ΔP+ΔP₁) liegt, und anschließend eine Aufbereitung (70) des Endoskops (2) durchgeführt wird, bei der Spülflüssigkeit unter Druck durch den wenigstens einen zu spülenden Kanal geleitet wird, wobei der pneumatische Überdruck im Endoskop (2) während der Aufbereitung innerhalb eines vorgebbaren oder vorgegebenen Luftdruckbereichs um das zweite Überdruckniveau (AP+ΔP₂) herum durch Hinzugeben oder Ablassen von Druckluft nachgeführt wird, **dadurch gekennzeichnet, dass** während der Aufbereitung (70) eine Differenz (84, ΔV) zwischen hinzugegebener und abgelassener Druckluft erfasst wird und bei einem Überwiegen des Ablassens von Druckluft gegenüber dem Hinzugeben von Druckluft über einen längeren Zeitraum während der Aufbereitung (70) und/oder nach einer Elimination eines Einflusses von Temperaturschwankungen eine Undichtigkeit des Endoskops (2) aufgrund eines Eindringens von Spülflüssigkeit aus einem zu spülenden Kanal in das Endoskop (2) festgestellt wird.

2. Verfahren nach Anspruch 1, **dadurch gekennzeichnet, dass** zur Eliminierung des Einflusses von Temperaturschwankungen die Menge an Druckluft berechnet wird, die zwischen zwei unterschiedlichen Zeitpunkten (64) hinzugekommen oder abgelassen worden ist, bei denen Temperaturen gemessen wurden, deren Unterschied weniger als 15 °C beträgt, insbesondere weniger als 3 °C.

3. Verfahren nach Anspruch 1, **dadurch gekennzeichnet, dass** zur Eliminierung des Einflusses von Temperaturschwankungen die Menge an hinzugekommener oder abgelassener Druckluft um den Effekt von Temperaturschwankungen korrigiert wird, wobei zur Korrektur insbesondere eine Musterfunktion verwendet wird, die anhand von in der vorausgegangenen Dichtigkeitsprüfung (50) ermittelten Faktoren an das Endoskop (2) angepasst wird.

4. Verfahren nach Anspruch 3, **dadurch gekennzeichnet, dass** die Musterfunktion eine lineare oder quadratische Funktion ist.

5. Verfahren nach einem der Ansprüche 1 bis 4, **dadurch gekennzeichnet, dass** die festgestellte Undichtigkeit des Endoskops (2) mit einem Grenzwert oder mehreren vorbestimmten oder vorbestimmbaren Grenzwerten verglichen wird.

6. Verfahren nach Anspruch 5, **dadurch gekennzeichnet, dass** bei einem Überschreiten eines ersten Grenzwerts der Überdruck (P) im Endoskop (2) auf oberhalb des zweiten Überdruckniveaus (AP+ΔP₂) erhöht wird.

7. Verfahren nach Anspruch 5 oder 6, **dadurch gekennzeichnet, dass** bei einem Überschreiten eines zweiten Grenzwerts, der insbesondere höher als der erste Grenzwert ist, die Aufbereitung (70) abgebrochen wird und insbesondere ein Defekt des Endoskops (2) festgestellt wird.

8. Vorrichtung (20) zum Beaufschlagen wenigstens eines aufzubereitenden, insbesondere flexiblen, Endoskops (2) mit Druckluft, die Teil eines Systems zum Aufbereiten wenigstens eines, insbesondere flexiblen, Endoskops ist, umfassend eine Druckluftquelle (22), einen an ein Endoskop (2) druckluftdicht anschließbaren Druckluftschlauch (23), eine mit dem Druckluftschlauch (23) direkt oder indirekt pneumatisch verbindbare Luftdruckmesseinheit (24) und eine mit der Druckluftquelle (22) und der Luftdruckmesseinheit (24) verbundene Steuereinheit (12), die ausgebildet und eingerichtet ist, in einem pneumatischen Dichtigkeitstest das Endoskop (2) durch den Druckluftschlauch (23) bis zu einem vorgegebenen oder vorgebbaren ersten Überdruckniveau (ΔP+ΔP₁) mit Druckluft zu beaufschlagen, während einer pneumatischen Dichtigkeitsmessung (50) ohne weitere Druckluftzufuhr einen Verlauf des Luftdrucks (P) im Endoskop (2) zu erfassen und aus einem Verlauf des Luftdrucks (P) während der pneumatischen Dichtigkeitsmessung (50) zu bestimmen, ob das Endoskop (2) den pneumatischen Dichtigkeitstest bestanden hat, nach Bestehen des pneumatischen Dichtigkeitstests einen Teil der Druckluft bis zum Erreichen eines zweiten Überdruckniveaus (AP+ΔP₂) aus dem Endoskop (2) abzulassen, das unterhalb des ersten Überdruckniveaus (ΔP+ΔP₁) liegt, und während einer Aufbereitung (70) des Endoskops (2), bei der Spülflüssigkeit unter Druck durch den wenigstens einen zu spülenden Kanal geleitet wird, den pneumatischen Überdruck im Endoskop (2) innerhalb eines vorgebbaren oder vorgegebenen Luftdruckbereichs um das zweite Überdruckniveau (AP+ΔP₂) herum durch Hinzugeben oder Ablassen von Druckluft nachzuführen, **dadurch gekennzeichnet, dass** die Steuereinrichtung (12) zusätzlich ausgebildet und eingerichtet ist, während der Aufbereitung (70) eine Differenz (84, ΔV) zwischen hinzugegebener und abgelassener Druckluft zu erfassen und bei einem Überwiegen des Ablassens von Druckluft gegenüber dem Hinzugeben von Druckluft über einen längeren Zeitraum während der Aufbereitung (70) und/oder nach einer Elimination des Einflusses von Temperaturschwankungen eine Undichtigkeit des Endoskops (2) aufgrund Eindringens von Spülflüssigkeit aus einem zu spülenden Kanal in das Endoskop (2) festzustellen.

9. Vorrichtung (20) nach Anspruch 8, **dadurch gekennzeichnet, dass** die Steuervorrichtung (12) ausgebildet und eingerichtet ist, ein Verfahren nach einem der Ansprüche 1 bis 7 durchzuführen, wobei die Steuervorrichtung (12) insbesondere auch eine Steuervorrichtung einer Aufbereitungseinheit (30) des Systems (10) zum Aufbereiten wenigstens eines, insbesondere flexiblen, Endoskops (2) ist.

10. System (10) zur Aufbereitung wenigstens eines, insbesondere flexiblen, Endoskops (2), umfassend eine Aufbereitungseinheit (30) und eine Vorrichtung (20) zum Beaufschlagen wenigstens eines aufzubereitenden, insbesondere flexiblen, Endoskops (2) mit Druckluft nach Anspruch 8 oder 9.
